# EUROPÄISCHE PATENTSCHRIFT

(11) **EP 1 410 783 B1**
(45) Veröffentlichungstag und Bekanntmachung des Hinweises auf die Patenterteilung: **21.06.2006**
(21) Anmeldenummer: 03027536.6
(22) Anmeldetag: 10.07.2002
(51) Int. Cl.: A61K 8/04, A61K 8/34, A61K 8/36, A61K 8/37, A61K 8/39, A61Q 17/04, A61Q 19/00

(54) **Schäumbare Zubereitungen**
Foaming compositions
Compositions moussantes

(30) Priorität: 17.07.2001 DE 10134597
(43) Veröffentlichungstag der Anmeldung: 21.04.2004
(62) Teilanmeldung aus: 02015388.8
(73) Patentinhaber: Beiersdorf AG, 20245 Hamburg (DE)
(72) Erfinder: Riedel, Heidi, 22529 Hamburg (DE); Kröpke, Rainer, 22869 Schenefeld (DE); Bleckmann, Andreas, 22926 Ahrensburg (DE); Oelrichs, Ilka, 25436 Tornesch (DE)

(56) Entgegenhaltungen:
- EP-A- 1 216 682
- EP-A- 1 216 683
- EP-A- 1 216 684
- FR-A- 2 745 496
- US-A- 4 808 388

## Beschreibung

Die vorliegende Erfindung betriff ein Verfahren zur Hersiellung von Schäumen ausgehend aus schäumbaren kosmetischen und dermatologischen Zube-<SHY>dermatologischen Zubereitungen, insbesondere hautpflegenden schäumbaren kosmetischen und dermatologischen Zubereitungen.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind - wie Emulsionen - thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüberhinaus oft wenig hautverträglich sind, zu erreichen.

Ein weiterer Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen.

Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterläßt einen Schaum. Derartige Systeme nach dem Stand der Technik entwicklen unter Zusatz von Treibgas ausschließlich wäßrig-feuchte Schäume, die nach der Applikation schnell brechen.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluß des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Eine Aufgabe der vorliegenden Erfindung war, den Stand der Technik zu bereichern und kosmetische oder dermatologische schäumbare Zubereitungen zur Verfügung zu stellen, die die Nachteile des Standes der Technik nicht aufweisen.

Das Dokument US-A-4808388 offenbart eine aus einem Spender-Flakon mittels Treibmitteln (N20 und/oder CO2) verschäumbare Creme-Emulsion zur Anwendung auf der Haut. Die Emulsion
Laut US-A-4808388 sind die Eigenschaften der aus der Creme-Emulsion erzeugenen Schäume deutlich verbessert, wenn - unabhängig vom Treibmittel - die in den Spender-Flakon einzufüllende und mit dem Treibmittel zu versetzende Creme-Emulsion bestimmte Zusammensetzungen und bestimmte physikalische Parameter aufweist, namlich die Zusammensetzungen 2,0- 9,0 Gew.-% nicht-ionische Netzmittel, 0,5 - 4,5 Gew.-% Konsistenzgeber, 4,5 - 21,0 Gew.-% Ölanteile, Rest ad 100 Gew.-% Wasser, enthallten und eine Viskosität von 200 - 500 mPas aufweisen.

Die Deutsche Offenlegungsschrift DE 197 54 659 offenbart, daß Kohlendioxid ein geeigneter Wirkstoff zur Stabilisierung oder Erhöhung der epidermalen Ceramidsyntheserate ist, welcher der Stärkung der Permeabilitätsbarriere, der Verminderung des transepidermalen Wasserverlusts und der Steigerung der relativen Hautfeuchtigkeit dienen kann. Zur Behandlung der Haut wird das CO₂ beispielsweise in Wasser gelöst, mit welchem anschließend die Haut gespült wird. Allerdings kennt der Stand der Technik bislang keinerlei kosmetische oder dermatologische Grundlagen, in die ein gasförmiger Wirkstoff in ausreichender, d. h. wirksamer Konzentration eingearbeitet werden könnte.

Es war überraschend und für den Fachmann nicht vorauszusehen, daß ein Verfahren zur Herstellung von Schaumen dadurch gekennzeichnet, daß schäumbare kosmetische oder dermatologische Zubereitungen, welche
I. ein Emulgatorsystem, welches aus
   A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
   besteht,
   und
II. bis zu 50 Gew.-% - bezogen auf das Gesamtgewicht der schäumbaren Zubereitung - einer Lipidphase, welche ein oder mehreren Lipide mit einer Polarität von höchstens 30 mN/m enthält,
enthalten, bei Entnahme aus einem Druckgasbehälter mit Hilfe von Kohlendioxid, Sauerstoff, Druckluft und/oder Stickstoff aufgeschäumt werden
den Nachteilen des Standes der Technik abhelfen.

Unter "schäumbar" ist im Sinne der vorliegenden Erfindung zu verstehen, daß die erfindungsgemäßen Zubereitungen Schäume bilden, wenn sie aufgeschäumt werden, d. h. wenn z. B. Gas in sie eingeblasen wird oder die Zubereitungen in der betreffenden Gasatmosphäre (heftig) geschlagen, geschüttelt, verspritzt oder gerührt werden. In derartig erzeugten Schäumen können die Gasbläschen (beliebig) verteilt in einer (oder mehreren) flüssigen Phase(n) vorliegen, wobei die Schäume makroskopisch nicht notwendigerweise das Aussehen eines Schaumes haben müssen.

Aus den oben genannten (o.g.) schäumbaren kosmetischen oder dermatologischen Zubereitungen können durch Aufschäumen z. B. makroskopisch sichtbar dispergierte Systeme aus in Flüssigkeiten dispergierten Gasen hergestellt werden. Der Schaumcharakter kann aber beispielsweise auch erst unter einem (Licht-) Mikroskop sichtbar werden. Darüber hinaus ist das Aufschäumen der o.g. schäumbaren Zubereitungen - insbesondere dann, wenn die Gasbläschen zu klein sind, um unter einem Lichtmikroskop erkannt zu werden - auch an einer starken Volumenzunahme des Systems erkennbar.

Die o.g. Zubereitungen stellen in jeglicher Hinsicht überaus befriedigende Präparate dar. Es war insbesondere überraschend, daß die aus den o.g. schäumbaren Zubereitungen hergestellten Schäume - auch bei einem ungewöhnlich hohen Gasvolumen - außerordentlich stabil sind. Dementsprechend eignen sich Zubereitungen im Sinne der vorliegenden Erfindung ganz besonders, um als Grundlage für Produktformen mit vielfältigen Anwendungszwecken zu dienen.

Die im vorliegen den Verfahren eingeseitzten Zubereitungen und daraus erhältliche Schäume zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege aus.

Aus der o.g. Zusammensetzungen sind feinblasige, reichhaltige Schäume von hervorragender kosmetischer Eleganz erhältlich. Weiterhin sind aus o.g. Zusammensetzungen besonders gut hautverträgliche Zubereitungen erhältlich, wobei wertvolle Inhaltsstoffe besonders gut auf der Haut verteilt werden können.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Butyloctanol, Butyldecanol, Hexyloctanol, Hexyldecanol, Octyldodecanol, Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 1 bis 20 Gew.-%, vorteilhaft von 2 bis 15 Gew.-%, insbesondere von 5 bis 10 Gew.-%, jeweils bezogen auf das Gesamtgewicht der Formulierung, zu wählen.

Die schäumbaren kosmetischen oder dermatologischen Mittel gemäß der Erfindung können beispielsweise aus Aerosolbehältern entnommen und dabei aufgeschäumt werden. Erfindungsgemäße Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Deratige Behälter können mit Ventilen sehr unterschiedlicher Bauart ausgestattet sein, die die Entnahme des Inhalts als Schaum ermöglichen.

Als Druckgasbehälter kommen im Sinne der vorliegenden Erfindung vor allem zylindrische Gefäße aus Metall (Aluminium, Weißblech, Inhalt <1000 mL), geschütztem bzw. nicht-splitterndem Glas oder Kunststoff (Inhalt < 220 mL) bzw. splitterndem Glas oder Kunststoff (Inhalt < 150 mL) in Frage, bei deren Auswahl Druck- und Bruchfestigkeit, Korrosionsbeständigkeit, leichte Füllbarkeit, ggf. Sterilisierbarkeit usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens. Für Glas- und Kunststoffdosen gelten niedrigere, von der Behältergröße und dem Treibmittel (ob verflüssigtes, verdichtetes oder gelöstes Gas) abhängige Werte für den Betriebsdruck.

Besonders vorteilhaft im Sinne der vorliegenden Erfindung sind Dosen aus Weißblech, Aluminium und Glas. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- sowie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt. Werden Kunststoffe als Sprüh-Behältermaterial verwendet, so sollten diese Chemikalien- und Sterilisationstemperatur-beständig, gasdicht, schlagfest und gegen Innendrücke über 12 bar stabil sein. Prinzipiell für Sprüh-Behälter-Zwecke geeignet sind Polyacetale und Polyamide.

Der innere Aufbau der Sprüh-Dosen sowie die Ventilkonstruktion sind je nach Verwendungs-Zweck und der physikalischen Beschaffenheit des Inhalts - z. B. ob als Zwei- oder als Dreiphasensystem - sehr variantenreich und können vom Fachmann durch einfaches Ausprobieren ohnen erfinderisches Zutun ermittelt werden. Für geeignete Ausführungsformen sei auf das "Aerosol Technologie Handbuch der Aersosol-Verpackung" hingewiesen *(Wolfgang Tauscher, Melcher Verlag GmbH Heidelberg*/*München,* 1996).

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:

| | |
|---|---|
| Teller: | Weißblech: blank, gold- bzw. klarlackiert, folienkaschiert (PE, PP oder PET) Aluminium: blank, silber- oder goldlackiert, verschiedene Lackvarianten, Stoner-Mudge-Ausführung |
| Dichtung: | natürliche bzw. synthetische Elastomere bzw. thermoplastische (Sleeve-Gaskets, folienkaschiert aus PE oder PP) Innen- und Aussendichtungen, z. B. aus Perbunan, Buna, Neopren, Butyl, CLB, LDPE, Viton, EPDM, Chlorbutyl, Brombutyl und/oder diversen Compounds |
| Kegel: | PA, POM, Messing sowie diversen Sondermaterialen, Standardbohrungen (z. B.: 0,25 bis 0,70 mm oder 2 x 0,45 bis 2 x 1,00 mm), verschiedene Schaftdurchmesser |
| Feder: | Metall, besonders bevorzugt V2A, rostfreier Stahl; Kunststoff und auch Elastomer |
| Gehäuse: | Standard und Impact VPH-Bohrungen, RPT-Bohrungen oder geschlitzt für Überkopf-Anwendungen Materialien: z. B. Polyacetal, PA, PE, POM und dergleichen mehr |
| Steigrohr: | Kunststoff (Polymer Resin), z.B. PE, PP, PA oder Polycarbonat |

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Auch Druckluft sowie weitere unter Druck befindliche Gase wie Luft, Sauerstoff, Stickstoff, und Kohlendioxid (CO₂) sind im Sinne der vorliegenden Erfindung als Treibgase (sowohl einzeln als in beleibigen Mischungen miteinander) zu verwenden.

Natürlich weiß der Fachmann, daß es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Clor, Brom, Iod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Besonders bevorzugtes Treibgas im Sinne der vorliegenden Erfindung ist Kohlendioxid. Insbesondere vorteilhaft sind aus erfindungsgemäßen Zubereitungen erhältliche Schäume, welche Kohlendioxid als einen oder den Wirkstoff enthalten. Ganz besonders vorteilhafte Schäume sind durch Aufschäumen der erfindungsgemäßen Zubereitungen mit Kohlendioxid, Sauerstoff, Druckluft und/oder Stickstoff erhältlich.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Vorteilhaft werden der oder die weiteren Emulgatoren aus der Gruppe gewählt, die die folgenden Verbindungen umfaßt:
Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Glycerylstearat SE, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium C₁₄₋₁₇ Alkyl Sec Sulfonat (Hostacerin CG von Hoechst), Glycerylstearat und PEG-100 Stearate (Arlacel 165 von ICI), Polysorbat 85, Trilaureth-4-Phosphat, PEG-35 Castor Oil, Sucrosestearat, Trioleth-8-Phosphat, C₁₂₋₁₅ Pareth-12, PEG-40 Hydrogenated Castor Oil, PEG-16 Soya Sterol, Polysorbat 80, Polysorbat 20, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, Glyceryllanolat, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Glycerylisostearat, Polyglyceryl-3-Diisostearate.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitans.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte selbstschäumende und/oder schäumbare Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Die Ölphase der erfindungsgemäßen Zubereitungen wird vorteilhaft gewählt aus der Gruppe der unpolaren Lipide mit einer Polarität ≤ 30 mN/m. Besonders vorteilhafte Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCI-Name** | **Polarität mN/m** |
|---|---|---|---|
| Stearinerie Dubois Fils | DUB VCI 10 | Isodecyl Neopentanoate | 29,9 |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Corning | DC Fluid 345 | Cyclomethicone | 28,5 |
| Dow Corning | Dow Corning Fluid 244 | Cyclopolydimethylsiloxan | 28,5 |
| Nikko Chemicals Superior Jojoba Oil Gold | Jojobaöl Gold | | 26,2 |
| Wacker | Wacker AK 100 | Dimethicone | 26,9 |
| ALZO (ROVI) | Dermol 98 | 2- Ethylhexanosäure 3,5,5 Trimethylester | 26,2 |
| Dow Corning | Dow Corning Fluid 246 | Offen | 25,3 |
| Henkel Cognis | Eutanol G | Octyldodecanol | 24,8 |
| Condea Chemie | Isofol 16 | Hexyl Decanol | 24,3 |
| ALZO (ROVI) | Dermol 139 | Isotridecyl 3,5,5 Trimethylhexanonanoate | 24,5 |
| Henkel Cognis | Cetiol PGL | Hexyldecanol (+) Hexyl Decyl Laurate | 24,3 |
| | Cegesoft C24 | Octyl Palmitate | 23,1 |
| Gattefosse | M.O.D. | Octyldodeceyl Myristate | 22,1 |
| | Macadamia Nut Oil | | 22,1 |
| Bayer AG, Dow Corning | Silikonöl VP 1120 | Phenyl Trimethicone | 22,7 |
| CONDEA Chemie | Isocarb 12 | Butyl Octanoicacid | 22,1 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Caprylate/Caprate | 21,5 |
| Unichema Huels | Miglyol 812 | Caprylic/Capric Triglyceride | 21,3 |
| Trivent (über S. Black) | Trivent OCG | Tricaprylin | 20,2 |
| ALZO (ROVI) | Dermol 866 | PEG " Diethylhexanoate/ Diisononanoate/ Ethylhexyl Isononanoate | 20,1 |
| Condea Chemie | Isofol 14 T | Butyl Decanol (+) Hexyl Octanol (+) Hexyl Decanol (+) Butyl Octanol | 19,8 |
| Lipochemicals INC. / USA (Induchem) | Lipovol MOS-130 | Tridecyl Stearate(+) Tridecyl Trimellitate(+) Dipentaerythrityl Hexacaprylate/Hexacaprate | 19,4 |
| | Ricinusoel | | 19,2 |
| CONDEA Chemie | Isofol Ester 0604 | | 19,1 |
| Huels CONDEA Chemie | Miglyol 840 | Propylene Glycol Dicaprylate/Dicaprate | 18,7 |
| CONDEA Chemie | Isofol 12 | Butyl Octanol | 17,4 |
| Goldschmidt | Tegosoft SH | Stearyl Heptanoate | 17,8 |
| | Avocadooel | | 14,5 |
| Henkel Cognis | Cetiol B | Dibutyl Adipate | 14,3 |
| ALZO (ROVI) | Dermol 488 | PEG 2 Diethylenhexanoate | 10,1 |
| Condea Augusta S.P.A. | Cosmacol ELI | C12-13 Alkyl Lactate | 8,8 |
| ALZO (ROVI) | Dermol 489 | Diethylen Glycol Dioctanoate(/ Diisononanoate | 8,6 |
| Condea Augusta S.P.A. | Cosmacol ETI | Di-C12113 Alkyl Tartrate | 7,1 |
| Henkel Cognis | Emerest 2384 | Propylene Glycol Monoisostearate | 6,2 |
| Henkel Cognis | Myritol 331 | Cocoglycerides | 5,1 |
| Unichema | Prisorine 2041 GTIS | Triisostearin | 2,4 |

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Die Gehalt der Lipidphase wird vorteilhaft kleiner als 50 Gew.-% gewählt, bevorzugt zwischen 1 und 40 Gew.-%, insbesondere bevorzugt zwischen 5 und 15 Gew.-%, jeweils bezogen auf das Gesamtgewicht der schäumbaren Zubereitung. Es ist gegebenenfalls ferner vorteilhaft, wenngleich nicht zwingend, wenn die Lipidphase bis zu 40 Gew.-% - bezogen auf das Gesamtgewicht der Lipidphase - an Lipiden mit einer Polarität ≥ 30 mN/m und/oder cyclischen oder linearen Silikonölen und/oder -wachsen enthält.

Vorteilhafte weitere Lipide im Sinne der vorliegenden Erfindung sind beispielsweise die im folgenden aufgelisteten:

| **INCI-Name** | **Polarität mN/m** |
|---|---|
| Cycloparaffin | 49,1 |
| Polydecene | 46,7 |
| Hydrogenated Polyisobutene | 44,7 |
| Polydimethylsiloxan | 46,5 |
| Isohexadecane | 43,8 |
| Mineral Oil | 43,7 |
| Mineral Oil | 43,7 |
| Polydimethylsiloxan | 42,4 |
| Isoeikosan | 41,9 |
| Polydimethylsiloxan | 40,9 |
| Ethoxydiglycol Oleate | 40,5 |
| Decyl Olivate | 40,3 |
| Dioctylcyclohexane | 39,0 |
| Mineral Oil | 38,3 |
| Paraffinum Liquidum | 37,6 |
| Isocetyl Palmitate | 36,2 |
| Cyclopentasiloxan | 32,3 |
| Octyl Isostearate | 31,6 |
| Dicaprylyl Carbonate | 31,7 |
| Trimethylhexyl Isononanoate | 31,1 |
| 2- Ethylhexyl Isononanoate | 31,0 |
| Octyl Cocoate | 30,0 |

Die erfindungsgemäßen kosmetischen und/oder dermatologischen Zubereitungen und daraus erhältliche Schäume können wie üblich zusammengesetzt sein. Besonders vorteilhaft im Sinne der vorliegenden Erfindung sind Zubereitungen zur Pflege der Haut: sie können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische oder topische dermatologische Zusammensetzungen im Sinne der vorliegenden Erfindung, beispielsweise verwendet werden als Hautschutzcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Ebenso wie Emulsionen von flüssiger und fester Konsistenz als kosmetische Reinigungslotionen bzw. Reinigungscremes Verwendung finden, können auch die aus erfindungsgemäßen Zubereitungen erhältlichen Schäume "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte "rinse off' Präparate angewendet werden, welche nach der Anwendung von der Haut abgespült werden

Aus erfindungsgemäßen kosmetischen und/oder dermatologischen Zubereitungen erhältliche Schäume können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Zur Anwendung werden die erfindungsgemäßen kosmetischen und dermatologischen Zubereitungen und/oder daraus erhältliche Schäume in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Farbstoffe, Pigmente, die eine färbende Wirkung haben, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A-palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäße Zubereitungen ist, daß diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z. B. Hydrocortison-17-valerat, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit® .

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

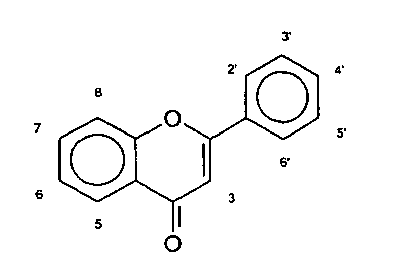

Einige der wichtigeren Flavone, welche auch bevorzugt in erfindungsgemäßen Zubereitungen eingesetz werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 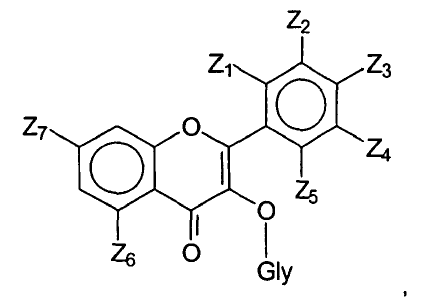 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 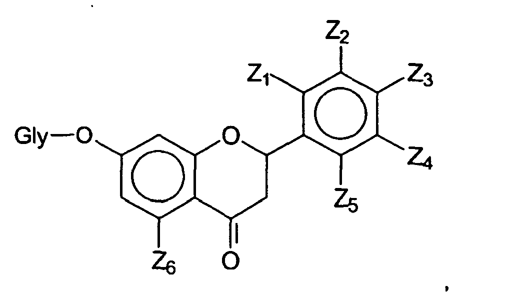 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 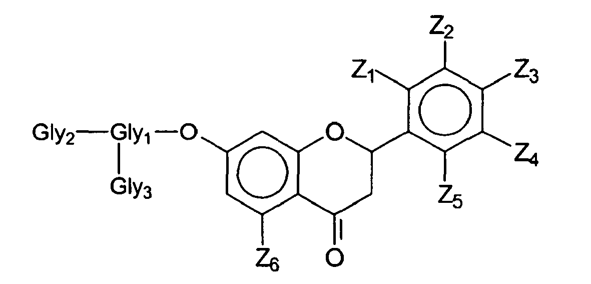 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

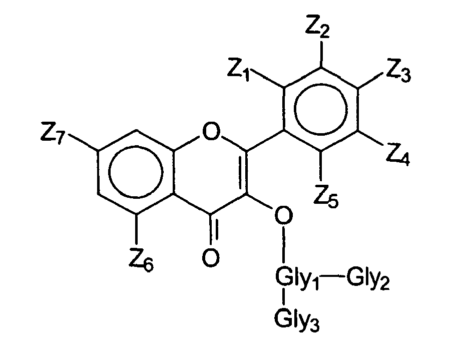

Besonders vorteilhaft werden die erfindungsgemäßen Flavonglycoside aus der Gruppe, welche durch die folgende Struktur wiedergegeben werden: 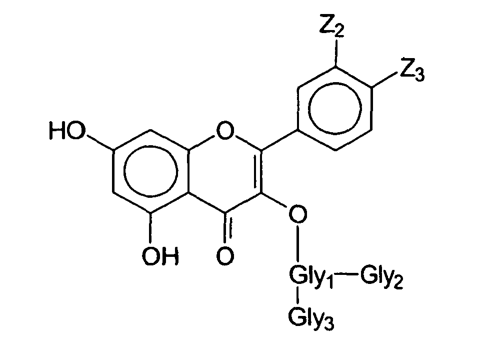 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomarein (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 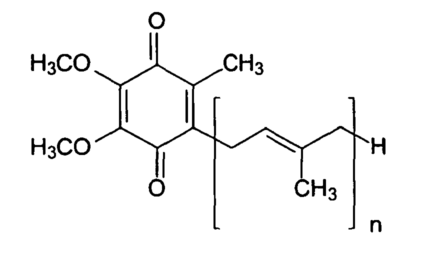 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

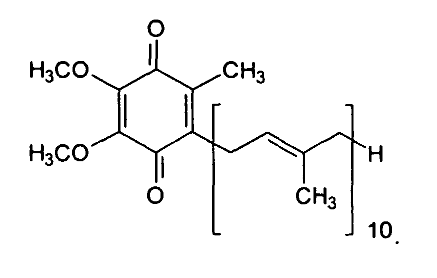

Plastochinone weisen die allgemeine Strukturformel 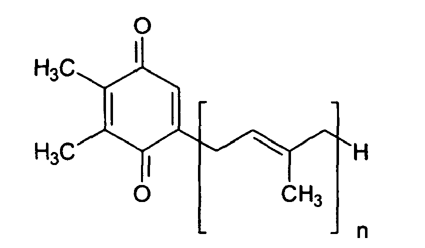 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

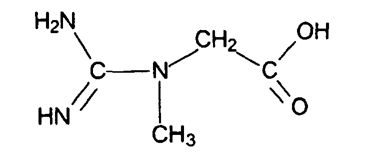

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Camitine, welche gewählt aus der Gruppe der Substanzen der folgenden allgemeinen Strukturformel 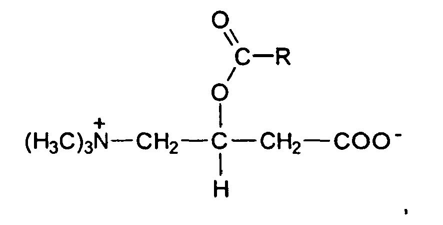 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Enantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Erstaunlicherweise können ausgewählte erfindungsgemäße Rezepturen auch eine Antifaltenwirkung aufweisen bzw. die Wirkung bekannter Antifaltenwirkstoffe erheblich steigern. Dementsprechend eignen sich Formulierungen im Sinne der vorliegenden Erfindung insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

In einer besonderen Ausführungsform betrifft die vorliegende Erfindung daher Produkte zur Pflege der auf natürliche Weise gealterten Haut, sowie zur Behandlung der Folgeschäden der Lichtalterung, insbesondere der unter a) bis g) aufgeführten Phänomene.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2-phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-Cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1' phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden. Auch Bariumsulfat (BaSO₄) ist vorteilhaft im Sinne der vorliegenden Erfindung.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete TiO₂-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 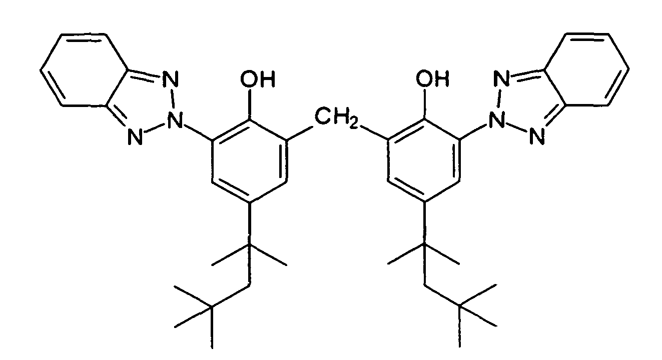 gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 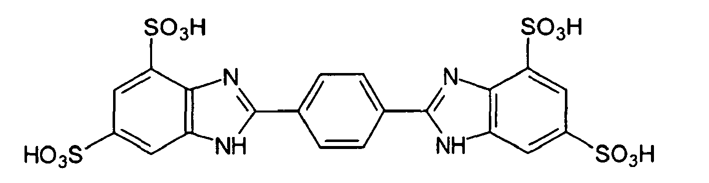 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 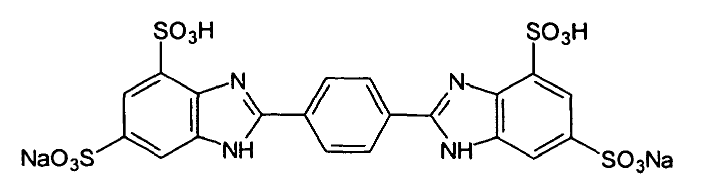 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

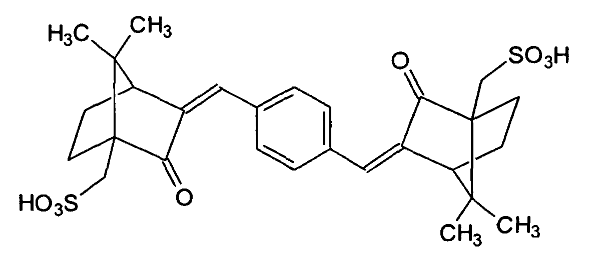

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 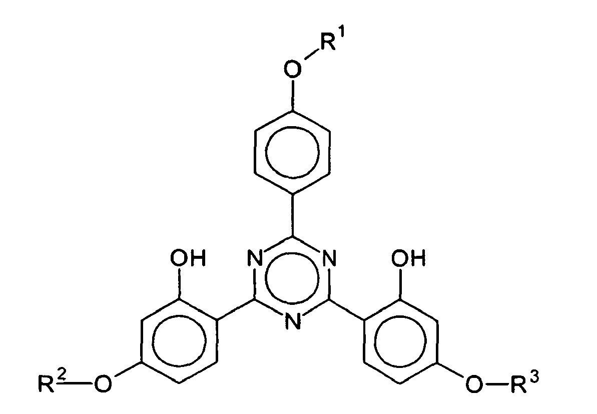 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 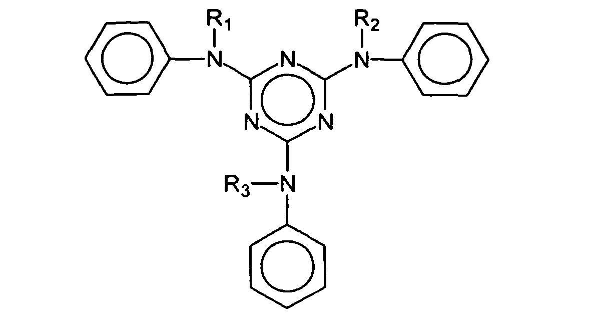 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 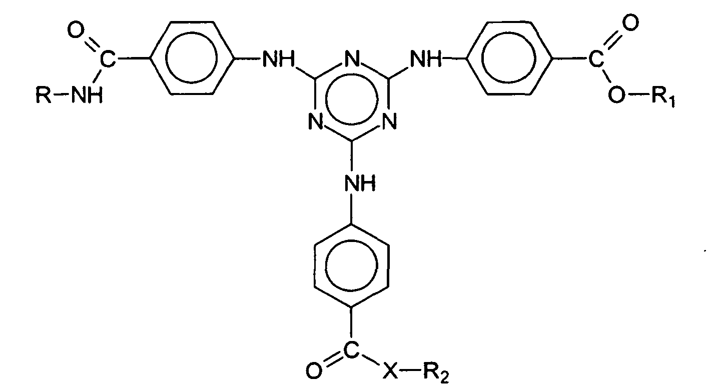 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 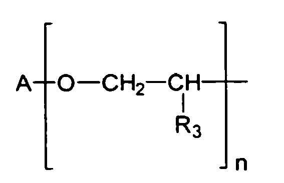 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 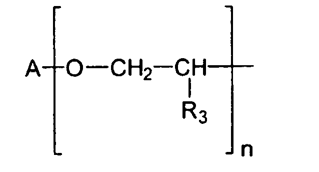 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt, wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 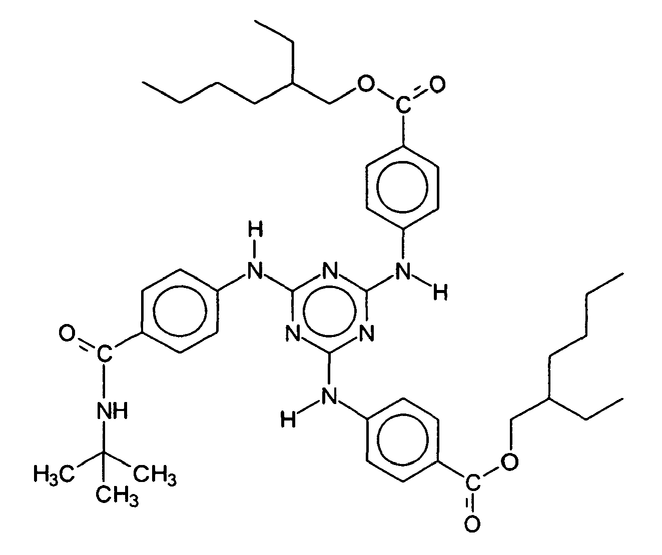 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoäsäure-tris(2-ethylhexyl-ester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 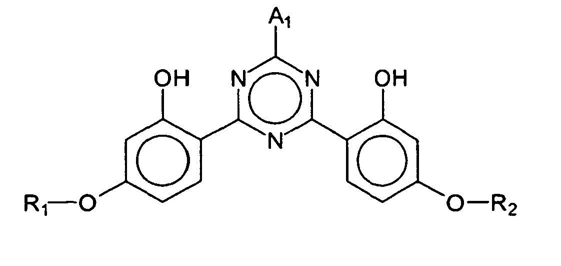 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl)-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 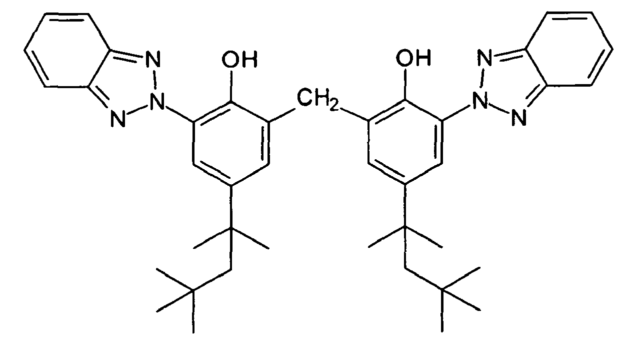 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 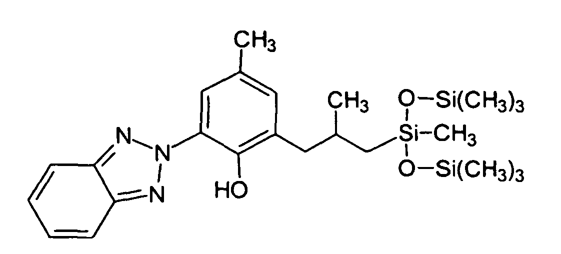 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

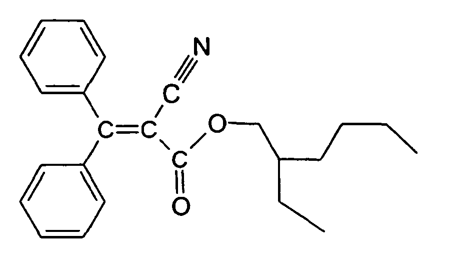

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### Vergleichs Beispiel 1 (schaumförmige O/W-Creme):

| **Emulsion I** | **Gew.-%** |
|---|---|
| Stearinsäure | 3,00 |
| Cetylalkohol | 8,50 |
| PEG-20 Stearat¹ | 8,50 |
| Stearyl Heptanoat | 4,00 |
| C12-13 Alkyl Lactat | 5,00 |
| Isohexadecan² | 2,00 |
| Glycerin | 5,00 |
| Natriumhydroxid | q.s. |
| Konservierung | q.s. |
| Parfum | q.s. |
| Wasser,demineralisiert | ad 100 |
| pH-Wert eingestellt auf 6,5-7,5 | |
| Zur Herstellung des Schaums werden 90 Vol.-% der Emulsion I mit 10 Vol.-% eines Treibgasgemisches aus Propan und Butan aufgeschäumt^{a}. | |

| | |
|---|---|
| ¹ Myrj 49, ICI Surfactants | |
| ² Solvent ICH, EC Erdölchemie Bayer AG | |

### Beispiel 2 (O/W-Lotion):

| **Emulsion II** | **Gew.-%** |
|---|---|
| Stearinsäure | 2,00 |
| Myristylalcohol | 1,50 |
| Cetylstearylalcohol | 0,50 |
| PEG-100 Stearat¹ | 3,0 |
| Mineralöl ² | 5,00 |
| Cocoglycerides | 15,0 |
| Glycerin | 3,00 |
| Natriumhydroxid | q.s. |
| Konservierung | q.s. |
| Parfum | q.s. |
| Wasser,demineralisiert | ad 100 |
| pH-Wert eingestellt auf 5,0-6,5 | |
| ^{a} Das Aufschäumen kann z. B. erfolgen, indem in die Zubereitungen Gas eingeblasen wird oder sie in der betreffenden Gasatmosphäre (heftig) geschlagen, geschüttelt, verspritzt oder gerührt werden. | |
| Zur Herstellung des Schaums werden 70 Vol.-% der Emulsion II mit 20 Vol.-% Kohlendi-oxid aufgeschäumt^{a}. | |

| | |
|---|---|
| Myrj 59p, ICI Surfactants, ² Hydrobrite 1000 PO, Witco BV, | |

### Vergleichs-Beispiel 3 (O/W-Lotion):

| **Emulsion III** | **Gew.-%** |
|---|---|
| Stearinsäure | 5,00 |
| Cetylstearylalkohol | 5,50 |
| PEG-30 Stearat¹ | 1,00 |
| Cyclomethicon² | 3,00 |
| Propylen Glykol Monoisostearat | 10,00 |
| Butyl Octanol | 10,00 |
| Citronensäure | 0,10 |
| Glycerin | 3,00 |
| Parfüm, Konservierungsmittel, | q.s. |
| Natriumhydroxid | q.s. |
| Farbstoffe usw. | q.s. |
| Wasser | ad 100 |
| pH-Wert eingestellt auf 6,0-7,5 | |
| Zur Herstellung des Schaums werden 95 Vol.-% der Emulsion III mit 5 Vol.-% eines Treibgasgemisches aus Propan und Butan aufgeschäumt^{a}. | |

| | |
|---|---|
| ¹ Myrj 51, ICI Surfactants, ² Dow Corning Fluid 245, Dow Corning | |

### Beispiel 4 (O/W-Emulsions-Make-up):

| **Emulsion IV** | **Gew.-%** |
|---|---|
| Palmitinsäure | 2,00 |
| Cetylalkohol | 2,00 |
| PEG-100 Stearat¹ | 2,00 |
| Dimethicon ² | 0,50 |
| Dibutyl Adipat | 10,00 |
| Dicaprylyl Ether³ | 1,50 |
| Glycerin | 3,00 |
| Glimmer | 1,00 |
| Eisenoxid | 1,00 |
| Titandioxid | 4,50 |
| Vitamin A Palmitat | 0,10 |
| Natriumhydroxid | q.s. |
| Konservierung | q.s. |
| Parfum | q.s. |
| Wasser,demineralisiert | ad 100 |
| pH-Wert eingestellt auf 6,0 -7,5 | |
| Zur Herstellung des Schaums werden 85 Vol.-% der Emulsion IV mit 15 Vol.-% Stickstoff aufgeschäumt^{a}. | |

| | |
|---|---|
| Myrj 59p, ICI Surfactants, ² Wacker Silikonöl AK 35, Wacker, ³ Cetiol OE, Henkel Cognis | |

### Vergleichs-Beispiel 5 (OIW-Creme):

| **Emulsion V** | **Gew.-%** |
|---|---|
| Stearinsäure | 4,00 |
| Cetylalkohol | 2,00 |
| PEG-30 Stearat¹ | 2,00 |
| Sorbitan Monostearat ² | 1,50 |
| Propylen Glykol Monoisostearat | 5,00 |
| Cyclomethicon ³ | 1,00 |
| Vitamin E Acetate | 1,00 |
| Retinylpalmitat | 0,20 |
| Glycerin | 3,00 |
| BHT | 0,02 |
| Disodium EDTA | 0,10 |
| Parfüm, Konservierungsmittel, Farbstoffe | q.s. |
| Kaliumhydroxid | q.s. |
| Wasser | ad 100 |
| pH-Wert eingestellt auf 5,0-7,0 | |
| Zur Herstellung des Schaums werden 89 Vol.-% der Emulsion V mit 11 Vol.-% Lachgas aufgeschäumt^{a}. | |

| | |
|---|---|
| Myrj 51, ICI Surfactants, ² Glycomol S, Akzo Nobel, ³ Dow Corning Fluid 245, Dow Corning | |

### Vergleichs-Beispiel 6 (O/W-Lotion):

| **Emulsion VI** | **Gew.-%** |
|---|---|
| Stearinsäure | 4,00 |
| Cetylstearylalkohol | 1,00 |
| PEG-100 Stearat¹ | 1,00 |
| Propylen Glykol Dicaprylat/ Dicaprat | 6,50 |
| Avocadoöl | 3,50 |
| Dimethicon ² | 2,50 |
| Vitamin E Acetat | 2,00 |
| Glycerin | 3,00 |
| Parfüm, Konservierungsmittel, Farbstoffe usw. | q.s. |
| Natriumhydroxid | q.s. |
| Wasser | ad 100 |
| pH-Wert eingestellt auf 6,0-7,5 | |
| Zur Herstellung des Schaums werden 92 Vol.-% der Emulsion VI mit 8 Vol.-% eines Treibgasgemisches aus Propan und Butan aufgeschäumt^{a}. | |

| | |
|---|---|
| ¹Myrj 59p, ICI Surfactants, ² Wacker Silikonöl AK 50, Wacker | |

### Vergleichs-Beispiel 7 (Sonnenschutz-Creme):

| **Emulsion VII** | **Gew.-%** |
|---|---|
| Stearinsäure | 1,00 |
| Cetylstearylalkohol | 4,00 |
| Myristylalkohol | 1,00 |
| PEG-20 Stearat¹ | 1,00 |
| Ricinusöl | 2,00 |
| Di-C12-13 Alkyl Tartrat | 16,00 |
| C12-15 Alkyl Benzoat | 3,50 |
| Dimethicon ² | 0,50 |
| Glycerin | 3,00 |
| Octylmethoxycinnamat ³ | 4,00 |
| Butylmethoxydibenzoylmethan ⁴ | 3,00 |
| Ethylhexyltriazon ⁵ | 3,00 |
| BHT | 0,02 |
| Disodium EDTA | 0,10 |
| Parfüm, Konservierungsmittel, Farbstoffe, usw. | q.s. |
| Kaliumhydroxid | q.s |
| Wasser | ad 100 |
| pH-Wert eingestellt auf 5,0-6,0 | |
| Zur Herstellung des Schaums werden 85 Vol.-% der Emulsion VII mit 15 Vol.-% Helium aufgeschäumt^{a}. | |

| | |
|---|---|
| ¹Myrj 49, ICI Surtactants, ² Wacker Silikonöl AK 50, Wacker, ³Escalol 5571, ISP-Van Dyke, ⁴Parsol 1789, Hoffmann La Roche, ⁵ Uvinul T150 | |

## Patentansprüche

1. Verfahren zur Herstellung von Schäumen **dadurch gekennzeichnet, daß** schäumbare kosmetische oder dermatologische Zubereitungen, welche
I. ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
besteht,
und
II. bis zu 50 Gew.-% - bezogen auf das Gesamtgewicht der schäumbaren Zubereitung - einer Lipidphase, welche ein oder mehrere polare Lipide mit einer Polarität von höchstens 30 mN/m enthält,
enthalten, bei Entnahme aus einem Druckgasbehälter mit Hilfe von Kohlendioxid, Sauerstoff, Druckluft und/oder Stickstoff aufgeschäumt werden.

2. Verfahren nach Anspruch 1, **dadurch gekennzeichnet, daß** die Lipidphase der schäumbaren Zubereitung bis zu 40 Gew.-% - bezogen auf das Gesamtgewicht der Lipidphase -Lipide mit einer Polarität von mindestens 30 mN/m und/oder cyclische oder lineare Silikonöle und/oder -wachse enthält.

3. Verfahren nach einem der vorhergehenden Ansprüche, **dadurch gekennzeichnet, daß** die Gewichtsverhältnisse von Emulgator A zu Emulgator B zu Coemulgator C (A : B : C) wie a : b : c gewählt wird, wobei a, b und c unabhängig voneinander rationale Zahlen von 1 bis 5, bevorzugt von 1 bis 3 darstellen.

4. Verfahren nach einem der vorhergehenden Ansprüche, **dadurch gekennzeichnet, daß** die Gewichtsverhältnisse von Emulgator A zu Emulgator B zu Coemulgator C (A : B : C) wie etwa 1 : 1 : 1 gewählt werden.

5. Verfahren nach einem der vorhergehenden Ansprüche, **dadurch gekennzeichnet, daß** die Gesamtmenge der Substanzen gemäß A., B. und C. aus dem Bereich von 1 bis 20 Gew.-%, bezogen auf das Gesamtgewicht der Formulierung, gewählt werden.

6. Verfahren nach einem der vorhergehenden Ansprüche, **dadurch gekennzeichnet, daß** die schäumbare Zubereitung weitere Emulgatoren, gewählt aus der Gruppe der hydrophilen Emulgatoren, insbesondere Mono-, Di-, Trifettsäureestem des Sorbitans, enthalten.

7. Verfahren nach Anspruch 6, **dadurch gekennzeichnet, daß** die Gesamtmenge der weiteren Emulgatoren kleiner als 5 Gew.-%, bezogen auf das Gesamtgewicht der Formulierung, gewählt wird.

8. Verfahren nach einem der vorhergehenden Ansprüche, **dadurch gekennzeichnet, daß** die schäumbare Zubereitung eine oder mehrere Substanzen, gewählt aus der Gruppe der Moisturizer, enthält.

9. Verfahren nach einem der vorhergehenden Ansprüche, **dadurch gekennzeichnet, daß** die schäumbare Zubereitung eine oder mehrere Substanzen, gewählt aus der Gruppe der UV-Filtersubstanzen, enthält.

10. Verfahren nach einem der vorhergehenden Ansprüche, **dadurch gekennzeichnet, daß** die Druckgasbehälter in Form zylindrischer Gefäße vorliegen.

11. Verfahren nach einem der vorhergehenden Ansprüche, **dadurch gekennzeichnet, daß** die Druckgasbehälter aus Metall oder aus nicht-splitterndem Glas oder Kunststoff bestehen.

12. Verfahren nach einem der vorhergehenden Ansprüche, **dadurch gekennzeichnet, daß** die Druckgasbehälter aus geschütztem, splitterndem Glas oder Kunststoff bestehen.

## Claims

1. Method of preparing foams **characterized in that** foamable cosmetic or dermatological preparations which comprise
I. an emulsifier system which consists of
A. at least one emulsifier A chosen from the group of completely neutralized, partially neutralized or unneutralized branched and/or unbranched, saturated and/or unsaturated fatty acids with a chain length of from 10 to 40 carbon atoms,
B. at least one emulsifier B chosen from the group of polyethoxylated fatty acid esters with a chain length of from 10 to 40 carbon atoms and a degree of ethoxylation of from 5 to 100 and
C. at least one coemulsifier C chosen from the group of saturated and/or unsaturated, branched and/or unbranched fatty alcohols with a chain length of from 10 to 40 carbon atoms,
and
II. up to 50% by weight - based on the total weight of the foamable preparation - of a lipid phase which comprises one or more polar lipids with a polarity of at most 30 mN/m,
are foamed upon removal from a pressurized-gas container with the help of carbon dioxide, oxygen, compressed air and/or nitrogen.

2. Method according to Claim 1, **characterized in that** the lipid phase of the foamable preparation comprises up to 40% by weight - based on the total weight of the lipid phase - of lipids with a polarity of at least 30 mN/m and/or cyclic or linear silicone oils and/or silicone waxes.

3. Method according to one of the preceding claims, **characterized in that** the weight ratios of emulsifier A to emulsifier B to coemulsifier C (A:B:C) is chosen as a:b:c, where a, b and c, independently of one another, are rational numbers from 1 to 5, preferably from 1 to 3.

4. Method according to one of the preceding claims, **characterized in that** the weight ratios of emulsifier A to emulsifier B to coemulsifier C (A:B:C) are chosen to be about 1:1:1.

5. Method according to one of the preceding claims, **characterized in that** the total amount of the substances according to A., B. and C. are chosen from the range from 1 to 20% by weight, based on the total weight of the formulation.

6. Method according to one of the preceding claims, **characterized in that** the foamable preparation comprises further emulsifiers chosen from the group of hydrophilic emulsifiers, in particular mono-, di-, tri- fatty acid esters of sorbitan.

7. Method according to Claim 6, **characterized in that** the total amount of the further emulsifiers is chosen to be less than 5% by weight, based on the total weight of the formulation.

8. Method according to one of the preceding claims, **characterized in that** the foamable preparation comprises one or more substances chosen from the group of moisturizers.

9. Method according to one of the preceding claims, **characterized in that** the foamable preparation comprises one or more substances chosen from the group of UV filter substances.

10. Method according to one of the preceding claims, **characterized in that** the pressurized-gas containers are in the form of cylindrical vessels.

11. Method according to one of the preceding claims, **characterized in that** the pressurized-gas containers consist of metal or of shatterproof glass or plastic.

12. Method according to one of the preceding claims, **characterized in that** the pressurized-gas containers consist of protected, shattering glass or plastic.

## Revendications

1. Procédé en vue de la fabrication de mousses, **caractérisé en ce que** des préparations cosmétiques ou dermatologiques, pouvant être mis sous forme de mousse, qui contiennent
I. un système d'émulsifiants qui se compose
A. d'au moins un émulsifiant A, sélectionné parmi le groupe des acides gras saturés et/ou insaturés, ramifiés et/ou non ramifiés, complètement, partiellement ou non neutralisés, ayant une longueur de chaîne de 10 à 40 atomes de carbone,
B. d'au moins un émulsifiant B, sélectionné parmi le groupe des esters d'acide gras polyéthoxylés, ayant une longueur de chaîne de 10 à 40 atomes de carbone et un degré d'éthoxylation de 5 à 100 et
C. d'au moins un coémulsifiant C, sélectionné parmi le groupe des alcools gras ramifiés et/ou non ramifiés, saturés et/ou insaturés, ayant une longueur de chaîne de 10 à 40 atomes de carbone,
et
II. jusqu'à 50% en poids, par rapport au poids total de la préparation pouvant être mise sous forme de mousse, d'une phase de lipides, qui contient un ou plusieurs lipides polaires ayant une polarité d'au plus 30 mN/m,
sont mis sous forme de mousse lors du prélèvement hors d'un cylindre à gaz comprimé à l'aide de dioxyde de carbone, d'oxygène, d'air comprimé et/ou d'azote.

2. Procédé selon la revendication 1, **caractérisé en ce que** la phase de lipides de la composition pouvant être mis sous forme de mousse contient jusqu'à 40% en poids - par rapport au poids total de la phase de lipides - des lipides ayant une polarité d'au moins 30 mN/m et/ou des huiles de silicone et/ou des cires cycliques ou linéaires.

3. Procédé selon l'une quelconque des revendications précédentes, **caractérisé en ce que** les rapports pondéraux de l'émulsifiant A à l'émulsifiant B au coémulsifiant C (A : B : C) sont choisis comme a : b : c, a, b et c représentant, indépendamment les uns des autres, des nombres rationnels allant de 1 à 5, de préférence, de 1 à 3.

4. Procédé selon l'une quelconque des revendications précédentes, **caractérisé en ce que** les rapports pondéraux de l'émulsifiant A à l'émulsifiant B au coémulsifiant C (A : B : C) sont choisis comme par exemple 1 : 1 : 1.

5. Procédé selon l'une quelconque des revendications précédentes, **caractérisé en ce que** les quantités totales des substances selon A., B. et C. sont choisies du domaine de 1 à 20 % en poids, par rapport au poids total de la formulation.

6. Procédé selon l'une quelconque des revendications précédentes, **caractérisé en ce que** la préparation pouvant être mise sous forme de mousse contient des émulsifiants supplémentaires, choisis parmi le groupe des émulsifiants hydrophiles, en particulier des esters des acides mono-, bi- et tri-gras de la sorbitane.

7. Procédé selon la revendication 6, **caractérisé en ce que** la quantité totale des émulsifiants supplémentaires est choisie inférieure à 5 % en poids, par rapport au poids total de la formulation.

8. Procédé selon l'une quelconque des revendications précédentes, **caractérisé en ce que** la préparation pouvant être mise sous forme de mousse contient un ou plusieurs substances, choisies parmi le groupe des agents hydratants.

9. Procédé selon l'une quelconque des revendications précédentes, **caractérisé en ce que** la préparation pouvant être mise sous forme de mousse contient une ou plusieurs substances, choisies parmi le groupe des agents de filtration des rayons UV.

10. Procédé selon l'une quelconque des revendications précédentes, **caractérisé en ce que** les cylindres à gaz comprimé sont présents sous la forme de récipients cylindriques.

11. Procédé selon l'une quelconque des revendications précédentes, **caractérisé en ce que** les cylindres à gaz comprimé sont constitués d'un métal ou d'un verre ou d'une matière synthétique ne pouvant pas se fracturer.

12. Procédé selon l'une quelconque des revendications précédentes, **caractérisé en ce que** les cylindres à gaz comprimé sont constitués d'un verre ou d'une matière synthétique protégé(e), pouvant se fracturer.
